# EUROPÄISCHE PATENTSCHRIFT

(11) **EP 4 662 098 B1**
(45) Veröffentlichungstag und Bekanntmachung des Hinweises auf die Patenterteilung: **21.10.2026**
(21) Anmeldenummer: 24700249.6
(22) Anmeldetag: 09.01.2024
(51) Int. Cl.: B60W 30/095, A61B 5/369, B60W 50/08, B60W 60/00, G06F 18/241, G06N 3/08, B60W 50/00

(54) **VERFAHREN ZUM GENERIEREN VON TRAININGSDATEN FÜR EINEN SOFTWAREAGENTEN, VERFAHREN ZUM TRAINIEREN EINES SOFTWAREAGENTEN, VERFAHREN ZUM ASSISTIEREN EINES FAHRERS BEIM STEUERN EINES KRAFTFAHRZEUGS SOWIE KRAFTFAHRZEUG**
METHOD FOR GENERATING TRAINING DATA FOR A SOFTWARE AGENT, METHOD FOR TRAINING A SOFTWARE AGENT, METHOD FOR ASSISTING A DRIVER IN THE CONTROL OF A MOTOR VEHICLE, AND MOTOR VEHICLE
PROCÉDÉ DE GÉNÉRATION DE DONNÉES D'ENTRAÎNEMENT POUR UN AGENT LOGICIEL, PROCÉDÉ D'ENTRAÎNEMENT D'UN AGENT LOGICIEL, PROCÉDÉ D'ASSISTANCE D'UN CONDUCTEUR DANS LA COMMANDE D'UN VÉHICULE AUTOMOBILE ET VÉHICULE AUTOMOBILE

(30) Priorität: 09.02.2023 DE 102023201074
(43) Veröffentlichungstag der Anmeldung: 17.12.2025
(73) Patentinhaber: VOLKSWAGEN AKTIENGESELLSCHAFT, 38440 Wolfsburg (DE)
(72) Erfinder: SCHACHER, Samuel, 10629 Berlin (DE); EL AMOURI, Amira, 38440 Wolfsburg (DE)
(86) Internationale Anmeldenummer: PCT/EP2024/050343
(87) Internationale Veröffentlichungsnummer: WO 2024/165251

(56) Entgegenhaltungen:
- EP-A1- 3 843 014
- DE-A1- 102010 063 792
- DE-A1- 102016 224 291
- DE-A1- 102020 202 231
- DE-A1- 102021 206 886
- DE-T5- 112019 000 582

## Beschreibung

Die Erfindung betrifft ein Verfahren zum Generieren von Trainingsdaten für einen Softwareagenten, ein Verfahren zum Trainieren eines Softwareagenten, ein Verfahren zum Assistieren eines Fahrers beim Steuern eines Kraftfahrzeugs mittels einer Fahrerassistenzfunktion sowie ein Kraftfahrzeug mit einem Fahrerassistenzsystem.

Die DE 10 2018 202 146 A1 offenbart ein Verfahren zum Auswählen eines Fahrprofils eines Kraftwagens, ein Fahrerassistenzsystem hierfür und einen damit ausgestatteten Kraftwagen. Es ist vorgesehen, dass anhand einer Bedienhandlung des Fahrers eine Abweichung zwischen dem aktuell von dem Fahrer gewünschten und dem bis dahin verwendeten, von mehreren vorgegebenen Fahrprofilen erkannt wird. Nach dem Erkennen der Abweichung wird eine Abfrage an den Fahrer ausgegeben, ob die Abweichung für die aktuelle Situation von dem Fahrerassistenzsystem angelernt werden soll. Nach dem Bestätigen der Abfrage durch den Fahrer wird das Fahrerassistenzsystem für die aktuelle Situation demgemäß in zumindest einem das Auswählen des zu verwendenden Fahrprofils beeinflussenden Parameter dahingehend angepasst, dass die Abweichung beim automatischen Auswählen des zu verwendenden Fahrprofils in zukünftigen Situationen, die der aktuellen Situation entsprechen, berücksichtigt wird.

Weiterhin offenbart die CN 109 927 725 A ein adaptives Geschwindigkeitsregelsystem mit der Fähigkeit, einen Fahrstil zu erlernen.

Darüber hinaus ist aus der EP 3 690 769 A1 ein Lernverfahren zum Erhalten mindestens einer personalisierten Belohnungsfunktion bekannt, welche zur Durchführung eines Reinforcement Learning Algorithmus verwendet wird und welche einer personalisierten optimalen Strategie für einen Fahrer entspricht.

Die DE 10 2020 202 231 A1 betrifft ein Verfahren zum Bereitstellen eines Trainingsdatensatzes zum Trainieren eines Maschinenlernverfahrens zum Schätzen einer Fahreraufmerksamkeit, wobei mittels mindestens eines Umfeldsensors erfasste Umfelddaten mindestens eines Fahrzeugs und/oder mittels mindestens eines Sensors erfasste und/oder abgefragte Fahrzeugdaten des mindestens einen Fahrzeugs empfangen werden, wobei auf Grundlage der erfassten Umfelddaten und/oder der erfassten und/oder abgefragten Fahrzeugdaten eine Situationskritikalität mittels eines Situationskritikalitätsmaßes bestimmt wird, wobei ein Labeling zumindest einer Teilmenge der erfassten Umfelddaten und/oder der erfassten und/oder abgefragten Fahrzeugdaten mit einer Fahreraufmerksamkeit ausgehend von der bestimmten Situationskritikalität durchgeführt wird, und wobei ein zumindest die Teilmenge umfassender Trainingsdatensatz zum Trainieren eines Maschinenlernverfahrens bereitgestellt wird.

Die DE 10 2016 224 291 A1 zeigt ein Verfahren zur rechnergestützten Adaption eines vorgegebenen teilautomatisierten Fahrsystems eines Kraftfahrzeugs, wobei das vorgegebene teilautomatisierte Fahrsystem eine Anzahl von Fahraufgaben eines Fahrers des Kraftfahrzeugs automatisch durchführt und selbsttätig in Grenzsituationen eine oder mehrere der Fahraufgaben an den Fahrer überträgt. Im Verfahren werden Trainingsdatensätze bereitgestellt, die von einer Anzahl von vorbestimmten teilautomatisierten Fahrsystemen erfasst wurden, welche jeweils eine Anzahl von Fahraufgaben durchführen, die den Fahraufgaben des vorgegebenen teilautomatisierten Fahrsystems entsprechen, und welche selbsttätig in Grenzsituationen eine oder mehrere der Fahraufgaben an den Fahrer übertragen. Ein jeweiliger Trainingsdatensatz zumindest eines Teils der Trainingsdatensätze spezifiziert eine aufgetretene Grenzsituation und die darauf folgende Reaktion des Fahrers. Mittels der Trainingsdatensätze wird das vorgegebene teilautomatisierte Fahrsystem basierend auf maschinellem Lernen trainiert.

Die DE 10 2021 206 886 A1 beschreibt ein Verfahren zum Betreiben eines Fahrerassistenzsystems eines Fahrzeugs, bei welchem das Fahrerassistenzsystem eine basierend auf einem Fahrweg wenigstens eines Fremdfahrzeugs erstellte Trajektorie heranzieht. Das Fahrerassistenzsystem nutzt die Trajektorie für eine Querführung des Fahrzeugs. Für das Bewerten der Trajektorie wird eine Reaktion eines Fahrers des Fahrzeugs auf ein Entlangfahren des Fahrzeugs entlang eines Teils der Trajektorie berücksichtigt.

Die EP 3 843 014 A1 zeigt ein Verfahren, Systeme und Vorrichtungen, einschließlich auf Datenträgern kodierter Computerprogramme, zur Generierung von Trainingsdaten für das Training eines Modells für maschinelles Lernen zur Trajektorienvorhersage. Ein Verfahren umfasst: die Gewinnung von Trainingsdaten, die Daten zur Charakterisierung eines Agenten in einer Umgebung zum Zeitpunkt der ersten Messung und Daten zur Charakterisierung einer möglichen Trajektorie des Agenten in der Umgebung für einen ersten Zeitraum nach der ersten Messung enthalten. Für die mögliche Trajektorie wird ein Langzeitlabel bestimmt, das angibt, ob der Agent ihr im ersten Zeitraum tatsächlich gefolgt ist. Außerdem wird ein Kurzzeitlabel bestimmt, das angibt, ob der Agent beabsichtigt hat, ihr zu folgen. Schließlich wird eine Referenzwahrscheinlichkeit für die mögliche Trajektorie ermittelt. Die Trainingsdaten werden mit der Referenzwahrscheinlichkeit für die mögliche Trajektorie verknüpft.

Ein Aspekt der DE 10 2010 063 792 A1 betrifft ein Verfahren zum automatischen und dynamischen Auswählen von Fahrerassistenzfunktionen. Jede der Fahrerassistenzfunktionen kann jeweils entweder einen aktivierten oder einen deaktivierten Zustand annehmen. Gemäß dem Verfahren wird fahrsituative Information über eine Fahrsituation ausgewertet, um eine automatische Auswahl zu erlauben. In Abhängigkeit der fahrsituativen Information wird eine Teilmenge aus der Mehrzahl der Fahrerassistenzfunktionen ausgewählt. Die Teilmenge der Fahrerassistenzfunktionen ist dann für die Fahrsituation im aktivierten Zustand zu betreiben.

Die DE 11 2019 000 582 T5 offenbart ein Verfahren zum Erzeugen einer künstlichen Intuitionsfunktionalität, die in der Lage ist, proaktiv bezüglich Situationen zu agieren, die ein erhöhtes Risiko für einen Verkehrsunfall umfassen. Das Verfahren umfasst: Erheben von Fahrzeugumgebungsdaten mittels zumindest eines Sensors in einem bemannten Fahrzeug; Erkennen einer vorbeugenden Fahrerhandlung/Reaktion, die ein erhöhtes Risiko für einen Verkehrsunfall anzeigt; Markieren der erhobenen Fahrzeugumgebungsdaten zu dem Zeitpunkt, zu dem die vorsorgliche Fahrerhandlung/Reaktion erkannt wird; Extrahieren erhobener Fahrzeugumgebungsdaten, die zum Lernen verwendet werden sollen, auf der Grundlage der markierten Fahrzeugumgebungsdaten; Erzeugen einer künstlichen Intuitionsfunktionalität, die in der Lage ist, proaktiv bezüglich Situationen zu agieren, die ein erhöhtes Risiko für einen Verkehrsunfall umfassen, indem ein auf Maschinenlernen beruhendes System trainiert wird, auf der Grundlage der extrahierten Fahrzeugumgebungsdaten; und Implementieren der erzeugten künstlichen Intuitionsfunktionalität in einer Steueranordnung eines autonomen Fahrzeugs.

Fahrerassistenzsysteme und Funktionen der automatisierten Fahrt übernehmen schrittweise Aufgaben des menschlichen Fahrens. Damit einhergehend bringen diese einen eigenen vorprogrammierten Fahrstil sowie eigene Fahrentscheidungen in die Fahraufgabe mit ein. Fahrstil beschreibt in diesem Kontext vorranging Aspekte die zwar auch durch äußere Randbedingungen wie Verkehrslage, Wetter und Zeit oder Fahrerfitness, beeinflusst werden, aber mehr den persönlichen Eigenschaften zuzuordnen sind, beispielsweise Geschwindigkeit- und Linienwahl, Beschleunigungs- und Bremsverhalten, Art wie durch einen Kreisverkehr gefahren wird, präferierter Abstand zum Rand, Ausrollen vor roten Ampeln oder Landstraßen-Orts-Einfahrten. Fahrentscheidungen beschreiben in diesem Kontext vorranging Aspekte des Überholens, von Spurwechseln, von Einfädelmanövern, dem allgemeinen Interagieren im Straßenverkehr sowie das Verhalten wie dicht man auffährt vor einer Aktion. Divergieren diese vorprogrammierten Eigenschaften von dem Fahrstil des Fahrers und den persönlichen beziehungsweise subjektiven Entscheidungen des nutzenden Fahrers, kann dies zu einer schlechten Bewertung bis hin zum Ausschalten beziehungsweise Nichtnutzen dieser Fahrerassistenzsysteme führen. Einer der größten Herausforderungen, insbesondere bei Fahrerassistenzsystemen aber auch bei Vollautomatisierung, ist es deswegen, dass der Kunde das System als positiv bewertet und Bereitschaft zeigt, es wirklich zu nutzen. Hier gibt es mehrere Stellhebel, wobei ein zentraler die Divergenz oder idealerweise nicht vorhandene Divergenz zwischen dem Fahrstil des Fahrers und den situativen Entscheidungen des Fahrerassistenzsystems ist.

Aufgabe der vorliegenden Erfindung ist es, eine Lösung zu schaffen, welche es ermöglicht, dass eine Fahrerassistenzfunktion besonders gut an einen Fahrer eines Kraftfahrzeugs angepasst wird.

Diese Aufgabe wird durch die Gegenstände der unabhängigen Ansprüche gelöst. Weitere mögliche Ausgestaltungen der Erfindung sind in den Unteransprüchen, der Beschreibung und den Figuren offenbart. Merkmale, Vorteile und mögliche Ausgestaltungen, die im Rahmen der Beschreibung für einen der Gegenstände der unabhängigen Ansprüche dargelegt sind, sind zumindest analog als Merkmale, Vorteile und mögliche Ausgestaltungen des jeweiligen Gegenstands der anderen unabhängigen Ansprüche sowie jeder möglichen Kombination der Gegenstände der unabhängigen Ansprüche, gegebenenfalls in Verbindung mit einem oder mehr der Unteransprüche, anzusehen.

Die Erfindung betrifft ein Verfahren zum Generieren von Trainingsdaten für einen Softwareagenten, welcher dazu eingerichtet ist, eine Fahrerassistenzfunktion eines Kraftfahrzeugs zu steuern. Mittels der Fahrerassistenzfunktion kann ein Fahrer des Kraftfahrzeugs in bestimmten Fahrsituationen unterstützt werden, beispielsweise durch Steuern einer Aktorik des Kraftfahrzeugs. Mittels der Fahrerassistenzfunktion kann der Fahrer beim Querführen und/oder beim Längsführen des Kraftfahrzeugs unterstützt werden.

Als Softwareagent, auch Agent oder Softbot, bezeichnet man ein Computerprogramm, das zu gewissem, spezifiziertem, eigenständigem und eigendynamischem Verhalten fähig ist. Das bedeutet, dass abhängig von verschiedenen Zuständen ein bestimmter Verarbeitungsvorgang abläuft, ohne dass von außen ein weiteres Startsignal gegeben wird oder während des Vorgangs ein äußerer Steuerungseingriff erfolgt. Eine Software kann als Agent definiert werden, wenn sie autonom, kognitiv, kommunikativ, modal adaptiv, aktiv, reaktiv, robust und/oder sozial ist. Diese Eigenschaften beschreiben einen Grad der Autonomie des Computerprogramms. Unter autonom ist zu verstehen, dass der Softwareagent unabhängig von Benutzereingriffen arbeitet. Unter kognitiv ist zu verstehen, dass der Softwareagent lernfähig ist und aufgrund zuvor getätigter Entscheidungen beziehungsweise Beobachtungen lernt. Unter kommunikativ ist zu verstehen, dass der Softwareagent seine Zustände als Wirkung auf seine Umgebung dieser mitteilt. Unter modal adaptiv ist zu verstehen, dass der Softwareagent aufgrund der eigenen Zustände und der Zustände der Umgebung seine eigenen Einstellungen insbesondere Parameter und/oder Struktur ändert. Unter aktiv ist zu verstehen, dass der Softwareagent Aktionen aufgrund eigener Initiative ausführt. Unter reaktiv ist zu verstehen, dass der Softwareagent auf Änderungen der Umgebung reagiert. Unter robust ist zu verstehen, dass der Softwareagent äußere und innere Störungen kompensiert. Unter sozial ist zu verstehen, dass der Softwareagent mit anderen Agenten kommuniziert.

Bei dem Verfahren ist es weiterhin vorgesehen, dass mittels der Fahrerassistenzfunktion für eine ermittelte Situation eine Trajektorie für das Kraftfahrzeug geplant wird. Weiterhin ist es vorgesehen, dass eine für das Führen des Kraftfahrzeugs entlang der Trajektorie vorgesehene Längssteuerung und/oder Quersteuerung geplant wird und für das Umsetzen der geplanten Längssteuerung und/oder Quersteuerung eine Aktorik des Kraftfahrzeugs eingestellt wird. Mit anderen Worten wird eine Bahnplanung mittels der Fahrerassistenzfunktion für das Kraftfahrzeug durchgeführt und die Aktorik des Kraftfahrzeugs, welche eine Lenkung und/oder Beschleunigung und/oder ein Abbremsen des Kraftfahrzeugs beeinflusst, dementsprechend angepasst, um das Kraftfahrzeug entlang der geplanten Trajektorie zu steuern beziehungsweise ein Führen des Kraftfahrzeugs entlang der Trajektorie zu assistieren. Bei dem Verfahren ist es weiterhin vorgesehen, dass eine Reaktion des Fahrers auf die eingestellte Aktorik ermittelt wird. Es wird somit festgestellt, wie der Fahrer auf die durch die Aktorik beeinflusste Lenkung und/oder Beschleunigung und/oder das durch die Aktorik beeinflusste Bremsen reagiert. In Abhängigkeit von der ermittelten Reaktion des Fahrers wird festgestellt, wie groß eine durch einen Divergenzwert charakterisierte Divergenz zwischen der durch die Reaktion charakterisierten, vom Fahrer geplanten Längssteuerung und/oder Quersteuerung und der durch die beeinflusste Aktorik charakterisierten, von der Fahrerassistenzfunktion geplanten Längssteuerung und/oder Quersteuerung des Kraftfahrzeugs entlang der Trajektorie ist. Es wird somit anhand der Reaktion des Fahrers auf die von der Fahrerassistenzfunktion eingestellte Aktorik festgestellt, ob der Fahrer mit der durch die eingestellte Aktorik charakterisierten, von der Fahrerassistenzfunktion geplanten Längssteuerung und/oder Quersteuerung einverstanden ist oder nicht einverstanden ist. Je geringer der Divergenzwert ist, desto kleiner ist die Abweichung zwischen der durch die Reaktion des Fahrers charakterisierten, von dem Fahrer geplanten Längssteuerung und/oder Quersteuerung des Kraftfahrzeugs und der durch die eingestellte Aktorik charakterisierten, von der Fahrerassistenzfunktion geplanten Längssteuerung und/oder Quersteuerung des Kraftfahrzeugs. Es kann somit bei einem geringen Divergenzwert davon ausgegangen werden, dass der Fahrer des Kraftfahrzeugs mit der durch die eingestellte Aktorik geplanten Längssteuerung und/oder Quersteuerung der Fahrerassistenzfunktion einverstanden ist. Bei einem hohen Divergenzwert wird davon ausgegangen, dass der Fahrer mit der durch die eingestellte Aktorik charakterisierten, von der Fahrerassistenzfunktion geplanten Längssteuerung und/oder Quersteuerung des Kraftfahrzeugs nicht einverstanden ist. Hierbei weicht die durch die Reaktion des Fahrers charakterisierte, von diesem geplante Längssteuerung und/oder Quersteuerung des Kraftfahrzeugs stark von der durch die eingestellte Aktorik charakterisierte, von der Fahrerassistenzfunktion geplanten Längssteuerung und/oder Quersteuerung des Kraftfahrzeugs ab. Der Divergenzwert charakterisiert somit inwieweit sich der Fahrer und die Fahrerassistenzfunktion hinsichtlich der geplanten Steuerung des Kraftfahrzeugs unterscheiden.

Bei dem Verfahren wird die Reaktion des Fahrers auf das durch die Fahrerassistenzfunktion assistierte Steuern des Kraftfahrzeugs ermittelt. Das bedeutet, dass in jedem Fall von der Fahrerassistenzfunktion die Aktorik des Kraftfahrzeugs angepasst wird. Ein Untersuchen sogenannter Ground Truth Daten, bei welchen das Kraftfahrzeug rein manuell von dem Fahrer gesteuert wird, ist nicht zwingend notwendig.

Bei dem Verfahren ist es weiterhin vorgesehen, dass die ermittelte Situation gemeinsam mit dem ermittelten zugeordneten Divergenzwert als Trainingsdaten für den Softwareagenten bereitgestellt wird. Bei dem Verfahren wird somit dem Fahrer des Kraftfahrzeugs beim Steuern des Kraftfahrzeugs mittels der Fahrerassistenzfunktion in der Situation assistiert, indem die Trajektorie für das Kraftfahrzeug geplant wird und in Abhängigkeit von der geplanten Trajektorie die Aktorik zur Quersteuerung und/oder Längssteuerung des Kraftfahrzeugs eingestellt wird. Bei dem Verfahren ist es vorgesehen, dass der jeweilige ermittelte Divergenzwert in jeweiligen ermittelten Situationen als sogenanntes Label den Trainingsdaten zugeordnet wird, sodass der Softwareagent besonders gut mittels der jeweiligen ermittelten Situationen auf gleiche oder ähnliche Situationen hin trainiert werden kann. Diese generierten Trainingsdaten ermöglichen, dass der Softwareagent derart trainiert wird, dass die durch den Divergenzwert charakterisierte Divergenz besonders gering ist. Das bedeutet, dass das Assistieren des Fahrers mittels der Fahrerassistenzfunktion besonders gut an jeweilige Wünsche beziehungsweise Erwartungen des Fahrers angepasst wird und als Zielwert gewählt wird, dass der Fahrer mit jeweiligen Aktionen der Fahrerassistenzfunktion einverstanden ist. Der Softwareagent kann beispielsweise ein künstliches neuronales Netz umfassen, welches mittels der generierten Trainingsdaten trainiert werden kann.

Zu der Erfindung kann weiterhin eine elektronische Recheneinrichtung gehören, welche dazu eingerichtet ist, das Verfahren zum Generieren des Trainingsdaten für den Softwareagenten durchzuführen. Die elektronische Recheneinrichtung kann dazu eingerichtet sein, die Fahrerassistenzfunktion zu steuern und/oder das Einstellen der Aktorik auszulösen. Weiterhin kann die elektronische Recheneinrichtung dazu eingerichtet sein, die Reaktion des Fahrers zu ermitteln und in Abhängigkeit von der ermittelten Reaktion wiederum die durch den Divergenzwert charakterisierte Divergenz zu ermitteln. Weiterhin kann die elektronische Recheneinrichtung dazu eingerichtet sein, aus der ermittelten Situation gemeinsam mit dem ermittelten zugeordneten Divergenzwert die Trainingsdaten zu generieren und diese für den Softwareagenten bereitzustellen.

In einer möglichen Weiterbildung des erfindungsgemäßen Verfahrens ist es vorgesehen, dass die Aktorik in Abhängigkeit von einer vorgegebenen Eingriffsdominanz der Fahrerassistenzfunktion eingestellt wird. Die Eingriffsdominanz charakterisiert, mit welcher Intensität die Fahrerassistenzfunktion die geplante, für die Trajektorie vorgesehene Längssteuerung und/oder Quersteuerung gegenüber Zielen und Aktionen des Fahrers durchsetzt. Die Eingriffsdominanz beschreibt eine Eingriffsstärke des Softwareagenten. Je höher die Eingriffsdominanz ist, desto stärker setzt die Fahrerassistenzfunktion die von der Fahrerassistenzfunktion geplante, entlang der Trajektorie vorgesehene Längssteuerung und/oder Quersteuerung gegenüber dem Fahrer durch. Bei einer Eingriffsdominanz von 0 % erfolgt ein manuelles Steuern des Kraftfahrzeugs durch den Fahrer. Bei einer Eingriffsdominanz von 100 % hat der Fahrer keinen Einfluss auf die Steuerung des Kraftfahrzeugs, sodass das Kraftfahrzeug rein von der Fahrerassistenzfunktion gesteuert wird. Hierbei wird der Fahrer von der Fahrerassistenzfunktion übersteuert. Bei einer mittleren Eingriffsdominanz wird dem Fahrer durch die Fahrerassistenzfunktion in unterschiedlichen Graden assistiert. Vorliegend ist die vorgegebene Eingriffsdominanz größer als 0 %. Die Einstellung der Aktorik wird je nach vorgegebener Eingriffsdominanz durch die Fahrerassistenzfunktion angepasst, um die von der Fahrerassistenzfunktion geplante, entlang der Trajektorie vorgesehene Längssteuerung und/oder Quersteuerung stärker oder weniger stark gegenüber Aktionen des Fahrers durchzusetzen. Beispielsweise kann die Aktorik bei einer geringen vorgegebenen Eingriffsdominanz für die Lenkung den Fahrer lediglich dadurch unterstützen, dass das Aufbringen eines Lenkmoments auf eine Lenkeinrichtung in eine Richtung vereinfacht und in die andere Richtung erschwert wird. Bei einer hohen Eingriffsdominanz kann durch die Fahrerassistenzfunktion die Lenkung des Kraftfahrzeugs durch Aufbringen eines Lenkmoments unmittelbar beeinflusst werden. Durch das Einstellen der Aktorik in Abhängigkeit von der vorgegebenen Eingriffsdominanz kann sichergestellt werden, dass ein vorgesehener Unterstützungsgrad des Fahrers durch die Fahrerassistenzfunktion eingehalten wird.

In einer weiteren möglichen Ausgestaltung der Erfindung ist es vorgesehen, dass die Reaktion des Fahrers anhand einer Betätigung eines Gaspedals und/oder eines Bremspedals und/oder einer Lenkeinrichtung ermittelt wird. Es wird somit die Reaktion des Fahrers anhand einer Beeinflussung der Fahrtrichtung und/oder der Beschleunigung des Kraftfahrzeugs und somit anhand der Beeinflussung der Längsführung beziehungsweise der Querführung des Kraftfahrzeugs durch den Fahrer ermittelt. Anhand dessen, wie sehr der Fahrer die Längsführung und/oder die Querführung des Kraftfahrzeugs beeinflusst, wird somit festgestellt, wie der Fahrer auf die Anpassung der Aktorik durch die Fahrerassistenzfunktion reagiert. Anhand der Betätigung des Gaspedals und/oder des Bremspedals und/oder der Lenkeinrichtung kann die Reaktion des Fahrers besonders einfach und zuverlässig ermittelt werden.

In einer weiteren möglichen Ausgestaltung der Erfindung ist es vorgesehen, dass die Reaktion des Fahrers anhand eines gemessenen fehlerbezogenen Hirnpotentials im Gehirn des Fahrers ermittelt wird. Erkennt eine Person einen Fehler während einer Aufgabe, dann kann als Reaktion das fehlerbezogene Hirnpotential im Gehirn dieser Person gemessen werden. Dieses fehlerbezogene Hirnpotential im Gehirn des Fahrers kann beispielsweise mittels einer Elektrodenkappe, welche der Fahrer auf dem Kopf trägt, gemessen werden. Das bedeutet, dass zum Ermitteln der Fahrerreaktion das im Gehirn des Fahrers bei dem durch die Fahrerassistenzfunktion assistierten Steuern des Kraftfahrzeugs in der ermittelten Situation gemessen wird. Mit anderen Worten wird bei dem Verfahren für die ermittelte Situation mittels der Fahrerassistenzfunktion die Aktorik des Kraftfahrzeugs eingestellt, um dem Fahrer des Kraftfahrzeugs beim Steuern des Kraftfahrzeugs zu assistieren, wobei zusätzlich das Hirnpotential im Gehirn des Fahrers ermittelt wird, um anhand des Hirnpotentials die Reaktion des Fahrers auf die Unterstützung durch die Fahrerassistenzfunktion festzustellen. Anhand des Hirnpotentials kann besonders gut erkannt werden, ob der Fahrer mit der Unterstützung der Querführung und/oder Längsführung durch die Fahrerassistenzfunktion einverstanden ist oder nicht einverstanden ist, ohne dass der Fahrer explizit die Querführung und/oder die Längsführung des Kraftfahrzeugs beeinflusst. Es kann somit anhand des Hirnpotentials besonders gut eine unbewusste Zustimmung des Fahrers beziehungsweise unbewusste Ablehnung des Fahrers für die Unterstützung der Längssteuerung und/oder Quersteuerung des Kraftfahrzeugs durch die Fahrerassistenzfunktion festgestellt werden.

In einer weiteren möglichen Ausgestaltung der Erfindung ist es vorgesehen, dass der Divergenzwert mit einem vorgegebenen Schwellenwert verglichen wird. Weiterhin ist es vorgesehen, dass in den Trainingsdaten der Situation zugeordnet wird, dass bei der Situation keine Divergenz besteht, wenn festgestellt wird, dass der Divergenzwert kleiner als der vorgegebene Schwellenwert ist. In den Trainingsdaten wird der Situation zugeordnet, dass eine Divergenz besteht, wenn festgestellt wird, dass der Divergenzwert größer als oder gleich wie der vorgegebene Schwellenwert ist. Das bedeutet, dass in den Trainingsdaten der jeweiligen Situation das Label zugeordnet sein kann, dass eine Divergenz besteht oder dass keine Divergenz besteht. Es kann somit in den Trainingsdaten für die jeweiligen Situationen ganz einfach gelabelt sein "Divergenz: ja" oder "Divergenz: nein". Dies ermöglicht, dass der Softwareagent mittels der Trainingsdaten besonders einfach trainiert wird.

Die Erfindung betrifft des Weiteren ein Verfahren zum Trainieren eines Softwareagenten, welcher insbesondere ein künstliches neuronales Netz umfasst, mittels Trainingsdaten, welche in einem Verfahren, wie es bereits im Zusammenhang mit dem erfindungsgemäßen Verfahren zum Generieren von Trainingsdaten beschrieben worden ist, generiert worden sind. Durch das Trainieren des Softwareagenten kann der Softwareagent die Fahrerassistenzfunktion besonders gut an einen jeweiligen Fahrerwunsch des Kraftfahrzeugs angepasst steuern. Hierdurch wird sichergestellt, dass eine Zustimmung des Fahrers zu einer Unterstützung der Längssteuerung und/oder Quersteuerung des Kraftfahrzeugs mittels der Fahrerassistenzfunktion besonders hoch ist. Infolgedessen ist eine Wahrscheinlichkeit dafür, dass der Fahrer des Kraftfahrzeugs die Fahrerassistenzfunktion beim Steuern des Kraftfahrzeugs nutzt, besonders hoch.

Erfindungsgemäß kann zudem eine elektronische Recheneinrichtung vorgesehen sein, welche dazu eingerichtet ist, das Verfahren zum Trainieren des Softwareagenten mittels der Trainingsdaten durchzuführen. Die elektronische Recheneinrichtung kann somit dazu eingerichtet sein, die generierten Trainingsdaten zu empfangen und den Softwareagenten zu trainieren. Bei der elektronischen Recheneinrichtung kann es sich beispielsweise um eine Hardwarekomponente handeln, auf welcher der Softwareagent ausgeführt werden kann.

In einer möglichen Weiterbildung des Verfahrens zum Trainieren des Softwareagenten ist es vorgesehen, dass der Softwareagent mittels bestärkendem Lernen, was auch als sogenanntes Reinforcement Learning bezeichnet wird, trainiert wird, indem eine Häufigkeit einer ermittelten Divergenz oder der Divergenzwert als Belohnung dient und zu minimieren ist. Das bedeutet, dass beim bestärkenden Lernen das Ziel gesetzt wird, dass der anhand der Reaktion des Fahrers auf die durch die Fahrerassistenzfunktion eingestellte Aktorik ermittelte Divergenzwert besonders niedrig ist. Alternativ oder zusätzlich kann für das bestärkende Lernen das Ziel gesetzt werden, dass bei mehreren durchgeführten Vorgängen in unterschiedlichen Situationen, bei welchen die Aktorik des Kraftfahrzeugs mittels der von dem Softwareagenten gesteuerten Fahrerassistenzfunktion angesteuert wird, eine Häufigkeit an jeweiligen Situationen, bei welchen eine Divergenz ermittelt worden ist, besonders gering ist im Vergleich zu einer Häufigkeit an Situationen, bei welchen keine Divergenz ermittelt worden ist. Das bedeutet, dass als Ziel für das bestärkende Lernen gesetzt wird, dass bei einer Vielzahl an Situationen, in welchen mittels der durch den Softwareagenten gesteuerten Fahrerassistenzfunktion der Fahrer des Kraftfahrzeugs beim Steuern des Kraftfahrzeugs unterstützt worden ist, ein Anteil an Situationen, bei welchen eine Divergenz ermittelt worden ist, besonders gering ist.

Bestärkendes Lernen, welches auch als verstärkendes Lernen bezeichnet wird, steht für eine Reihe von Methoden des maschinellen Lernens, bei denen der Softwareagent selbstständig eine Strategie erlernt, um erhaltene Belohnungen zu maximieren. Vorliegend wird die Belohnung maximiert, wenn ein besonders geringer Divergenzwert erreicht wird beziehungsweise ein besonders geringer Anteil an Situationen vorliegt, bei welchen jeweils eine Divergenz ermittelt worden ist. Bei dem bestärkenden Lernen wird dem Softwareagenten nicht vorgezeigt, welche Aktion und welche Situation die beste ist, sondern der Softwareagent erhält durch eine Interaktion mit seiner Umwelt zu bestimmten Zeitpunkten eine Belohnung, die auch negativ sein kann. Vorliegend kann der Softwareagent, sofern dieser beim Steuern des Kraftfahrzeugs trainiert wird, ständig mit neuen generierten Trainingsdaten trainiert und somit weiter verbessert werden. Das Verfahren des bestärkenden Lernens ermöglicht, dass die durch den Softwareagenten gesteuerte Fahrerassistenzfunktion besonders schnell und besonders gut an jeweilige Wünsche des Fahrers des Kraftfahrzeugs angepasst wird.

Die Erfindung betrifft des Weiteren ein Verfahren zum Assistieren eines Fahrers beim Steuern eines Kraftfahrzeugs mittels einer Fahrerassistenzfunktion. Bei dem Verfahren wird durch einen Softwareagenten, welcher in einem Verfahren trainiert worden ist, wie es bereits im Zusammenhang mit dem erfindungsgemäßen Verfahren zum Trainieren eines Softwareagenten beschrieben worden ist, die Fahrerassistenzfunktion angepasst. Mittels der angepassten Fahrerassistenzfunktion wird dem Fahrer beim Steuern des Kraftfahrzeugs assistiert, indem mittels der Fahrerassistenzfunktion für eine ermittelte Situation eine Aktorik des Kraftfahrzeugs eingestellt wird. Es handelt sich dabei um einen "end-to-end"-Machine-Learning-Ansatz, bei denen die Situation darstellende Kameradaten dem Softwareagenten zur Verfügung gestellt werden und von dem Softwareagenten direkt Aktorikbefehle für das Kraftfahrzeug ausgegeben werden. Das Verfahren ermöglicht, dass das Kraftfahrzeug mit der besonders gut an Wünsche des Fahrers angepassten Fahrerassistenzfunktion gesteuert werden kann.

In einer möglichen Weiterbildung des Verfahrens ist es vorgesehen, dass mittels des Softwareagenten eine Bahnplanung der Fahrerassistenzfunktion und/oder eine Eingriffsdominanz der Fahrerassistenzfunktion angepasst wird. Mittels der angepassten Fahrerassistenzfunktion wird dem Fahrer beim Steuern des Kraftfahrzeugs assistiert, indem mittels der Fahrerassistenzfunktion für eine ermittelte Situation eine Trajektorie für das Kraftfahrzeug im Rahmen der angepassten Bahnplanung geplant wird, eine für das Führen des Kraftfahrzeugs entlang der Trajektorie vorgesehene Längssteuerung und/oder Quersteuerung geplant wird und für das Umsetzen der geplanten Längssteuerung und/oder Quersteuerung eine Aktorik des Kraftfahrzeugs in Abhängigkeit von der angepassten Eingriffsdominanz eingestellt wird. Der Softwareagent kann hierbei selbst die Fahrerassistenzfunktion durchführen oder die Fahrerassistenzfunktion kann durch ein Fahrerassistenzsystem durchgeführt werden, zu welchem der Softwareagent unterschiedlich ist und welches von dem Softwareagenten zum Anpassen der Bahnplanung und/oder der Eingriffsdominanz beeinflusst und somit geändert werden kann. Das Anpassen der durch die Fahrerassistenzfunktion durchgeführten Bahnplanung und/oder Eingriffsdominanz mittels des trainierten Softwareagenten ermöglicht, dass die Fahrerassistenzfunktion beziehungsweise die vom Fahrer beim Steuern des Kraftfahrzeugs von der Fahrerassistenzfunktion erhaltene Unterstützung besonders gut an einen Fahrerwunsch des Fahrers angepasst werden kann.

In diesem Zusammenhang kann es insbesondere vorgesehen sein, dass eine Kritikalität der ermittelten Situation festgestellt wird und die Eingriffsdominanz in Abhängigkeit von der festgestellten Kritikalität der Situation gewählt wird. Das bedeutet, dass je kritischer die Situation eingeschätzt wird, desto größer wird die Eingriffsdominanz der Fahrerassistenzfunktion gewählt. Wird somit festgestellt, dass eine kritische Situation besteht, dann greift die Fahrerassistenzfunktion durch Einstellen der Aktorik besonders stark in die Längsführung und/oder Querführung des Kraftfahrzeugs ein, um eine Gefahr für das Kraftfahrzeug und Fahrzeuginsassen des Kraftfahrzeugs abzuwenden oder zumindest abzumildern. Wird festgestellt, dass die ermittelte Situation besonders wenig kritisch ist, dann kann die Eingriffsdominanz besonders niedrig gewählt werden, sodass die Querführung und/oder Längsführung des Kraftfahrzeugs besonders wenig von der Fahrerassistenzfunktion beeinflusst wird. Es wird dem Fahrer somit bei dieser besonders wenig kritischen Situation besonders freie Hand beim Längsführen und/oder Querführen des Kraftfahrzeugs gelassen. Hierdurch kann dem Fahrer das Gefühl gegeben werden, dass er das Steuern des Kraftfahrzeugs besonders gut beeinflussen kann und somit eine besonders große Kontrolle über die Steuerung des Kraftfahrzeugs hat.

Die Erfindung betrifft des Weiteren ein Kraftfahrzeug, mit einem Fahrerassistenzsystem, welches dazu eingerichtet ist, einem Fahrer beim Steuern des Kraftfahrzeugs mittels einer Fahrerassistenzfunktion zu assistieren. Diese Fahrerassistenzfunktion ist in einem Verfahren, wie es bereits im Zusammenhang mit dem erfindungsgemäßen Verfahren zum Assistieren eines Fahrers beim Steuern eines Kraftfahrzeugs mittels einer Fahrerassistenzfunktion beschrieben worden ist, angepasst worden. Dieses Fahrerassistenzsystem kann dazu eingerichtet sein, von dem trainierten Softwareagenten angepasst zu werden. Alternativ kann der Softwareagenten das Fahrerassistenzsystem oder ein Teil davon sein und dazu eingerichtet sein, die Fahrerassistenzfunktion unmittelbar zu steuern.

Weitere Merkmale der Erfindung können sich aus der nachfolgenden Figurenbeschreibung sowie anhand der Zeichnung ergeben.

Die Zeichnung zeigt in:
- Fig. 1: ein erstes Verfahrensschema für ein Verfahren zum Generieren von Trainingsdaten für einen Softwareagenten sowie für ein Verfahren zum Assistieren eines Fahrers beim Steuern eines Kraftfahrzeugs mittels einer Fahrerassistenzfunktion;
- Fig. 2: ein zweites Verfahrensschema für ein Verfahren zum Generieren von Trainingsdaten für einen Softwareagenten sowie für ein Verfahren zum Assistieren eines Fahrers beim Steuern eines Kraftfahrzeugs mittels einer Fahrerassistenzfunktion;
- Fig. 3: ein Koordinatensystem, in welchem eine tatsächlich gefahrene Trajektorie eines Kraftfahrzeugs dargestellt ist in einem Zustand, in welchem dem Fahrer des Kraftfahrzeugs mittels einer Fahrerassistenzfunktion beim Steuern des Kraftfahrzeugs assistiert worden ist, wobei zusätzlich eine von der Fahrerassistenzfunktion für das Kraftfahrzeug geplante Trajektorie eingezeichnet ist; und
- Fig. 4: ein weiteres Koordinatensystem, in welchem dargestellt ist, wie sich die von der Fahrerassistenzfunktion für das Kraftfahrzeug geplante Trajektorie von der tatsächlich von dem Kraftfahrzeug gefahrenen Trajektorie aus Fig. 3 unterscheidet.

Gleiche oder funktionsgleiche Elemente sind in den Figuren mit den gleichen Bezugszeichen versehen.

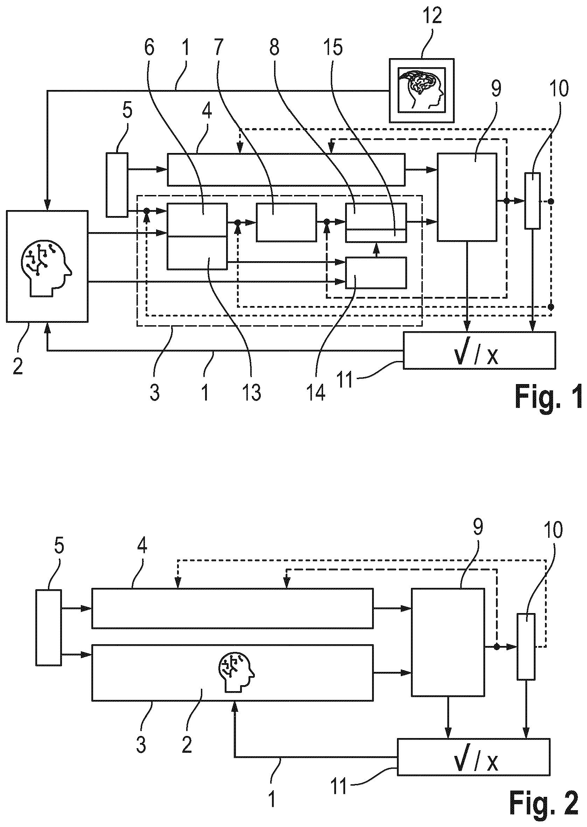

In Fig. 1 ist ein Verfahrensschema für ein Verfahren zum Generieren von Trainingsdaten 1 für einen Softwareagenten 2 gezeigt, welcher dazu eingerichtet ist, eine Fahrerassistenzfunktion 3 eines Kraftfahrzeugs zu steuern. Weiterhin ist in dem Verfahrensschema ein Verfahren zum Assistieren eines Fahrers 4 beim Steuern eines Kraftfahrzeugs mittels der Fahrerassistenzfunktion 3 dargestellt. Die Trainingsdaten 1 können generiert werden, während dem Fahrer 4 beim Steuern des Kraftfahrzeugs assistiert wird. In Fig. 2 ist ebenfalls ein Verfahrensschema für ein Verfahren zum Generieren von Trainingsdaten 1 für den Softwareagenten 2 gezeigt, wobei der Softwareagent 2 die Fahrerassistenzfunktion 3 ausführt und somit steuert. In dem Verfahrensschema in Fig. 2 ist weiterhin das Verfahren zum Assistieren des Fahrers 4 beim Steuern des Kraftfahrzeugs mittels der Fahrerassistenzfunktion 3 umfasst. Der Softwareagent 2 kann dazu eingerichtet sein, wie in Fig. 1 gezeigt ist, die Fahrerassistenzfunktion 3 anzupassen. Alternativ kann der Softwareagent 2, wie in Fig. 2 gezeigt ist, selbst die Fahrerassistenzfunktion 3 ausführen. Wie anhand der Verfahrensschemata in Fig. 1 und Fig. 2 erkannt werden kann, kann zum einen dem Fahrer 4 beim Steuern des Kraftfahrzeugs mittels der Fahrerassistenzfunktion 3 assistiert werden und gleichzeitig können die Trainingsdaten 1 für den Softwareagenten 2 generiert werden.

Um dem Fahrer 4 beim Steuern des Kraftfahrzeugs zu assistieren wird eine Situation 5 ermittelt, in welcher sich das Kraftfahrzeug befindet. Diese ermittelte Situation 5 wird für die Fahrerassistenzfunktion 3 bereitgestellt. Mittels der Fahrerassistenzfunktion 3 erfolgt im Rahmen einer Bahnplanung 6 ein Planen einer Soll-Trajektorie 16 für das Kraftfahrzeug für die ermittelte Situation 5. Anschließend wird mittels der Fahrerassistenzfunktion 3 eine Fahrdynamikregelung 7 durchgeführt im Rahmen von welcher eine für das Führen des Kraftfahrzeugs entlang der Soll-Trajektorie 16 vorgesehene Längssteuerung und/oder Quersteuerung geplant wird. Hierbei können beispielsweise Lenkwinkel für die geplante Soll-Trajektorie 16 berechnet werden. Die Fahrerassistenzfunktion 3 ist weiterhin dazu eingerichtet, anschließend eine Aktorikregelung 8 durchzuführen. Im Rahmen der Aktorikregelung 8 erfolgt eine Umsetzung des berechneten Lenkwinkels mittels Aktorik des Kraftfahrzeugs. Im Rahmen der Aktorikregelung 8 wird für das Umsetzen der geplanten Längssteuerung und/oder Quersteuerung die Aktorik des Kraftfahrzeugs eingestellt. In Folge des Anpassens der Aktorik kann eine Beeinflussung der Quer- und/oder Längssteuerung des Kraftfahrzeugs erfolgen, indem jeweilige Steuereinrichtungen 9 des Kraftfahrzeugs beeinflusst werden. Gleichzeitig kann im Kopf des Fahrers 4 eine Vorstellung davon bestehen, wie die Trajektorie auszusehen hat, entlang welcher das Kraftfahrzeug in der ermittelten Situation 5 zu führen ist und wie eine Längsführung des Kraftfahrzeugs entlang dieser Trajektorie auszusehen hat. Infolgedessen kann der Fahrer 4 die Quer- und/oder Längsführung des Kraftfahrzeugs beeinflussen. Diese Beeinflussung der Quer- und/oder Längsführung des Kraftfahrzeugs durch den Fahrer 4 führt wiederum zu einer von dem Kraftfahrzeug unter einem definierten Geschwindigkeitsprofil zurückgelegten Ist-Trajektorie 10. Anhand der in dem Kraftfahrzeug resultierenden Einstellung von Steuereinrichtungen 9, wie einem Gaspedal und/oder einem Bremspedal und/oder einer Lenkung sowie anhand der vom Kraftfahrzeug in der ermittelten Situation 5 tatsächlich gefahrenen Ist-Trajektorie 10 kann ermittelt werden, ob eine Divergenz 11 zwischen der von der Fahrerassistenzfunktion 3 geplanten Längssteuerung und/oder Quersteuerung des Kraftfahrzeugs entlang der Soll-Trajektorie 16 und einem Fahrerwunsch besteht. Diese Divergenz 11 wird in Abhängigkeit von einer Reaktion des Fahrers 4 auf die durch die Fahrerassistenzfunktion 3 vorgenommene Aktorikregelung 8 ermittelt.

Im Rahmen der Aktorikregelung 8 kann beispielsweise von der Fahrerassistenzfunktion 3 durch Anpassen jeweiliger Aktoren eingestellt werden, dass für den Fahrer 4 ein Drehen des Lenkrads in eine erste Richtung erleichtert und in eine zu der ersten Richtung entgegengesetzte zweite Richtung erschwert wird. Hierdurch kann mittels der Fahrerassistenzfunktion 3 der Fahrer 4 dazu bewegt werden, in die erste Richtung zu lenken, sodass das Kraftfahrzeug möglichst entsprechend der von der Fahrerassistenzfunktion 3 geplanten Soll-Trajektorie 16 geführt wird. Zum Assistieren des Fahrers 4 bei der Längssteuerung kann mittels der Fahrerassistenzfunktion 3 wenigstens ein Aktor beispielsweise derart eingestellt werden, dass dem Fahrer 4 ein Herunterdrücken des Gaspedals besonders leicht fällt und beim Drücken des Bremspedals ein Widerstand auftritt, um den Fahrer 4 dazu zu bringen, schneller entlang der Trajektorie zu fahren, indem der Fahrer 4 das Kraftfahrzeug beschleunigt. Durch die Aktorikregelung 8 kann somit die Fahrerassistenzfunktion 3 den Fahrer 4 dazu bringen, das Kraftfahrzeug entsprechend der von der Fahrerassistenzfunktion 3 geplanten Längsführung und/oder Querführung zu steuern. Durch entsprechendes Betätigen des Lenkrads beziehungsweise des Gaspedals beziehungsweise des Bremspedals kann der Fahrer 4 das Kraftfahrzeug entsprechend der von der Fahrerassistenzfunktion 3 geplanten Längsführung und/oder Querführung steuern oder der von der Fahrerassistenzfunktion 3 geplanten Längsführung und/oder Querführung entgegenwirken.

Für das Ermitteln der Divergenz 11 wird die Reaktion des Fahrers 4 auf die von der Fahrerassistenzfunktion 3 eingestellte Aktorik ermittelt. Hierbei wird die Reaktion des Fahrers 4 vorliegend in Abhängigkeit von der Betätigung der Steuereinrichtungen 9 durch den Fahrer 4 und/oder anhand der von dem Kraftfahrzeug in der ermittelten Situation 5 zurückgelegten Ist-Trajektorie 10 ermittelt. Dabei kann die Divergenz 11 beispielsweise in Abhängigkeit von einer Abweichung der von dem Kraftfahrzeug tatsächlich gefahrenen Ist-Trajektorie 10 zu der von der Fahrerassistenzfunktion 3 geplanten Soll-Trajektorie 16 ermittelt werden. Die Divergenz 11 kann insbesondere in Form eines Divergenzwerts, welcher die Divergenz 11 1charakterisiert, ermittelt werden. Die Divergenz 11 beschreibt die Abweichung zwischen der geplanten Längssteuerung und/oder Quersteuerung durch die Fahrerassistenzfunktion 3 im Vergleich zu der geplanten Längssteuerung und/oder Quersteuerung des Fahrers 4.

Bei dem Verfahren wird somit in Abhängigkeit von der ermittelten Reaktion des Fahrers 4 festgestellt, wie groß die durch den Divergenzwert charakterisierte Divergenz 11 der durch die Reaktion charakterisierten, vom Fahrer 4 geplanten Längssteuerung und/oder Quersteuerung zu der durch die beeinflusste Aktorik charakterisierten, von der Fahrerassistenzfunktion 3 geplanten Längssteuerung und der Quersteuerung des Kraftfahrzeugs entlang der geplanten Soll-Trajektorie 16 ist. Die Situation 5 wird gemeinsam mit dem ermittelten zugeordneten Divergenzwert als Trainingsdaten 1 für den Softwareagenten 2 bereitgestellt. Der Softwareagent 2 kann wiederum mittels der Trainingsdaten 1 trainiert werden, insbesondere mittels bestärkendem Lernen. Bei diesem Trainingsverfahren ist als Ziel vorgegeben, dass ein jeweiliger für weitere Situationen 5 ermittelte Divergenzwert besonders niedrig sein soll beziehungsweise dass für weitere untersuchte Situationen 5 möglichst selten ermittelt werden soll, dass eine Divergenz 11 besteht.

Vorliegend ist es vorgesehen, dass die Reaktion des Fahrers 4 zusätzlich anhand eines gemessenen fehlerbezogenen Hirnpotentials 12 ermittelt wird. Dieses Hirnpotential 12 kann mittels am Kopf des Fahrers 4 befestigten Elektroden ermittelt werden. Insbesondere wird ermittelt, wie das Hirnpotential 12 des Fahrers 4 auf die tatsächliche Längsführung und/oder Querführung des Kraftfahrzeugs in der ermittelten Situation 5 reagiert. Anhand des ermittelten fehlergemessenen Hirnpotentials 12 kann wiederum die durch den Divergenzwert charakterisierte Divergenz 11 bestimmt werden. Die Situation 5 kann gemeinsam mit dem anhand des Hirnpotentials 12 ermittelten Divergenzwert als Trainingsdaten 1 für den Softwareagenten 2 bereitgestellt werden. Die fehlerbezogenen Hirnpotentiale, welche auch als Error Potentials bezeichnet werden können, generiert das Gehirn automatisch, wenn die reale Welt von den eigenen Erwartungen abweicht. Diese können durch ein Brain-Computer-Interface gemessen werden. Macht die Fahrerassistenzfunktion 3 nicht das was der Fahrer 4 erwartet, dann kann das passive, aber messbare Error Potential Signal gemessen werden.

Im Rahmen der Bahnplanung 6 kann eine Sicherheitsvorhersage 13 getroffen werden. Im Rahmen dieser Sicherheitsvorhersage 13 wird eine Kritikalität der Situation 5 in Kombination mit der im Rahmen der Bahnplanung 6 ermittelten Soll-Trajektorie 16 bestimmt. Das bedeutet, dass ermittelt wird, wie wahrscheinlich eine Gefahr einer Beschädigung, insbesondere einer Kollision des Kraftfahrzeugs mit einem weiteren Objekt, in der Situation 5 ist und ob diese Gefährdung abgewandt werden kann, wenn das Kraftfahrzeug der im Rahmen der Bahnplanung 6 erstellten Soll-Trajektorie 16 folgt. In Abhängigkeit von dieser ermittelten Kritikalität der Situation 5 wird eine Eingriffsdominanz 14 der Fahrerassistenzfunktion 3 angepasst. Diese Eingriffsdominanz 14 bestimmt, wie stark die Steuerung des Kraftfahrzeugs von der Fahrerassistenzfunktion 3 und wie stark die Steuerung des Kraftfahrzeugs von dem Fahrer 4 zu beeinflussen sein soll. In Abhängigkeit von der eingestellten Eingriffsdominanz 14 kann die von der Aktorikregelung 8 eingestellte Aktorik mittels einer Anpassungsregelung 15 angepasst werden, wodurch die Längsführung und/oder Querführung des Kraftfahrzeugs entsprechend der Höhe der Eingriffsdominanz 14 mehr oder weniger von der Fahrerassistenzfunktion 3 beeinflusst wird.

Bei dem in Fig. 1 gezeigten Verfahren ist es vorgesehen, dass mittels des trainierten Softwareagenten 2 die Bahnplanung 6, die Sicherheitsvorhersage 13 und/oder die Eingriffsdominanz 14 angepasst werden. Durch das Anpassen der Sicherheitsvorhersage 13 kann der Softwareagent 2 vorgeben, welche Arten von Situationen 5 wie kritisch einzustufen sind. Über das Beeinflussen der Eingriffsdominanz 14 kann mittels des Softwareagenten 2 eingestellt werden, bei welcher ermittelten Kritikalität jeweiliger Situationen 5 der Fahrerassistenzfunktion 3 welche Dominanz gegenüber dem Fahrer 4 eingeräumt werden soll.

Mittels des Softwareagenten 2, welcher anhand der Trainingsdaten 1 trainiert worden ist, werden somit die Bahnplanung 6 und/oder die Eingriffsdominanz 14 der Fahrerassistenzfunktion 3 angepasst. Mittels der angepassten Fahrerassistenzfunktion 3 wird dem Fahrer 4 anschließend beim Steuern des Kraftfahrzeugs assistiert. Bei diesem Assistieren wird mittels der Fahrerassistenzfunktion 3 für die ermittelte Situation 5 eine Soll-Trajektorie 16 für das Kraftfahrzeug im Rahmen der angepassten Bahnplanung 6 geplant, eine für das Führen des Kraftfahrzeugs entlang der Soll-Trajektorie 16 vorgesehene Längssteuerung und/oder Quersteuerung geplant und für das Umsetzen der geplanten Längssteuerung und/oder Quersteuerung die Aktorik des Kraftfahrzeugs mittels der Aktorikregelung 8 in Abhängigkeit von der angepassten Eingriffsdominanz 14 eingestellt.

Um in den Trainingsdaten 1 den jeweiligen Situationen 5 besonders einfache Label zuzuordnen, kann es vorgesehen sein, dass der die Divergenz 11 charakterisierende ermittelte Divergenzwert mit einem vorgegebenen Schwellenwert verglichen wird und in den Trainingsdaten 1 der Situation 5 zugeordnet wird, dass bei der Situation 5 keine Divergenz 11 besteht, wenn festgestellt wird, dass der Divergenzwert kleiner als der vorgegebene Schwellenwert ist. Wird festgestellt, dass der Divergenzwert größer als oder gleich groß wie der vorgegebene Schwellenwert ist, dann wird in den Trainingsdaten 1 der Situation 5 zugeordnet, dass eine Divergenz 11 besteht. In den Trainingsdaten 1 ist somit für die jeweilige Situation 5 lediglich gelabelt "Divergenz: ja" oder "Divergenz: nein".

Bei der in Fig. 2 gezeigten Ausgestaltung des Verfahrens ist der Softwareagent 2 dazu eingerichtet, die Fahrerassistenzfunktion 3 auszuführen. Dabei wird der Softwareagent 2 analog zu dem bereits im Zusammenhang mit Fig. 1 beschriebenen Verfahren anhand der Trainingsdaten 1 trainiert, in welchen jeweiligen ermittelten Situationen 5 die zugehörige ermittelte Divergenz 11 in Form des Divergenzwerts oder in Form von "Divergenz besteht"/"Divergenz besteht nicht" als Label zugeordnet ist. Bei dem in Fig. 2 gezeigten Verfahren ist es vorgesehen, dass mittels des Softwareagenten 2 die Bahnplanung 6, die Fahrdynamikregelung 7 und die Aktorikregelung 8 durchgeführt werden. Zusätzlich können mittels des Softwareagenten 2 die Sicherheitsvorhersage 13 und das Anpassen der Eingriffsdominanz 14 sowie das Anpassen der durch die Aktorikregelung 8 vorgegebenen Aktorik in Abhängigkeit von der Eingriffsdominanz 14 mittels der Anpassungsregelung 15 durchgeführt werden.

Das Verfahren zum Assistieren des Fahrers 4 beim Steuern des Kraftfahrzeugs kann beispielsweise im Rahmen eines Spurhalteassistenten als Fahrerassistenzfunktion 3 verwendet werden. Mittels der Fahrerassistenzfunktion 3 kann somit ein Lenkmoment aufgebracht werden, wobei das Lenken des Kraftfahrzeugs durch den Fahrer 4 vorgenommen wird.

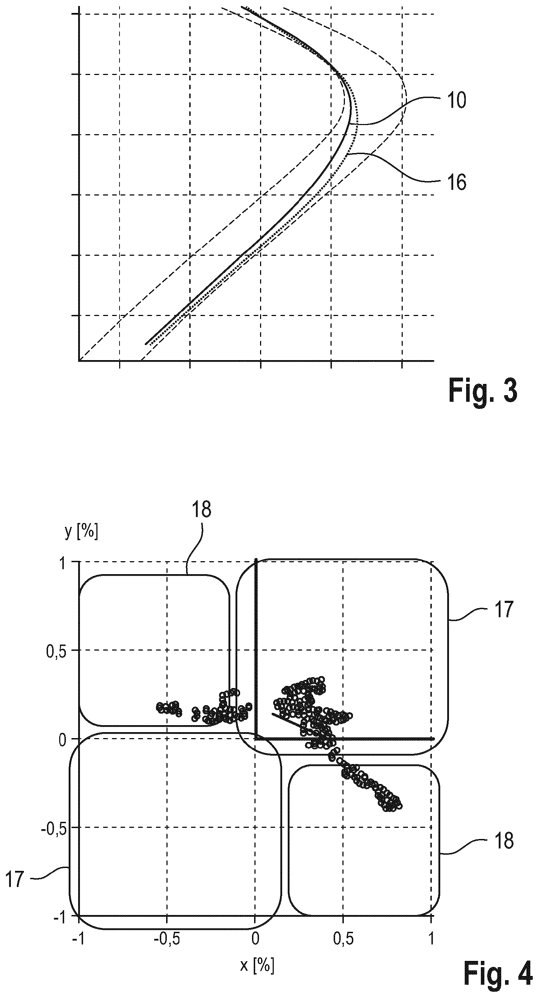

In Fig. 3 ist über den Verlauf einer Kurve die von dem Kraftfahrzeug gefahrene Ist-Trajektorie 10 im Vergleich mit der von der Fahrerassistenzfunktion 3 geplanten Soll-Trajektorie 16 dargestellt. Hierbei kann erkannt werden, dass die tatsächlich von dem Kraftfahrzeug gefahrene Ist-Trajektorie 10 von der geplanten Soll-Trajektorie 16 abweicht. Es besteht somit eine Divergenz 11 zwischen der durch die Soll-Trajektorie 16 ausgedrückten Planung der Quersteuerung des Kraftfahrzeugs durch die Fahrerassistenzfunktion 3 und der von dem Fahrer 4 gewünschten Querführung des Kraftfahrzeugs, welche in Kombination mit der durch das Fahrerassistenzfunktion 3 eingestellten Aktorik in der Ist-Trajketorie 10 resultiert ist.

In Fig. 4 ist an der Ordinatenachse das von der Fahrerassistenzfunktion 3 aufgebrachte Assistenzmoment y aufgetragen und an der Abszissenachse ist das durch den Fahrer 4 aufgebrachte Fahrermoment x in Prozent aufgetragen, wobei die jeweiligen Momente in Prozent und normalisiert zum Wert der Eingabe und somit normalisiert auf den Messbereich des jeweiligen das Moment erfassenden Sensors angegeben sind. In dem Koordinatensystem liegen zwei Konvergenzbereiche 17 sowie zwei Divergenzbereiche 18 vor. Die Konvergenzbereiche 17 charakterisieren Situationen, in welchen von dem Fahrer 4 ein gleiches oder ähnliches Moment aufgebracht wird wie von der Fahrerassistenzfunktion 3. In den jeweiligen Divergenzbereichen 18 weichen die von dem Fahrer 4 aufgebrachten Momente von den von der Fahrerassistenzfunktion 3 aufgebrachten Momenten ab. Je nachdem, ob die zu einem Zeitpunkt an den jeweiligen Sensoren gemessenen Messwerte einen Punkt in dem Diagramm ergeben, welcher sich in einem der Divergenzbereiche 18 oder in einem der Konvergenzbereiche 17 befindet, kann festgestellt werden, dass die Divergenz 11 besteht, wenn der Punkt in einem der Divergenzbereiche 18 liegt, und festgestellt werden, dass keine Divergenz 11 besteht, wenn der Punkt in einem der Konvergenzbereiche 17 liegt. In diese Korrelationsanalyse zwischen Fahrermoment x und Assistenzmoment y kann zusätzlich eine Abweichung der Ist-Trajektorie 10 zu der von der Fahrerassistenzfunktion 3 geplanten Soll-Trajektorie 16 miteinbezogen werden. Hierbei kann insbesondere eine Abweichungsrichtung miteinbezogen werden.

Eine Divergenz 11 zwischen dem Fahrer 4 und der Fahrerassistenzfunktion 3 tritt insbesondere bei der Bahnplanung 6 und/oder bei der Aktorikregelung 8 auf. Im Rahmen des Verfahrens wird ein in physischen Signalen messbarer Konflikt gemessen und zur Anpassung der Fahrerassistenzfunktion 3 genutzt. Im Rahmen der Bahnplanung 6 sowie der Aktorikregelung 8 können viele Konflikte beispielsweise durch abweichende Planungen oder durch ungünstige Eingriffsstrategien entstehen. Die Divergenzen 11 können beispielsweise entstehen, wenn der Fahrer 4 eine Handlung der Fahrerassistenzfunktion 3 nicht gut findet oder nicht erwartet hat oder die Fahrerassistenzfunktion 3 trotz einer gleichen geplanten Trajektorie diese in den Augen des Fahrers 4 komisch umgesetzt hat, beispielsweise zu steif.

Als zusätzliche statistische Analysemöglichkeit für die Bewertung der Divergenz 11 können ein Mittelwert sowie eine Kovarianzmatrix verwendet werden, insbesondere für Supervised Learning Ansätze für nachträgliche Analyse und Adaption oder als Episodenreward für einen Reinforcement Learning Agenten.

Seit Einführung von Assistenzsystemen wird daran gefeilt, wie eine Divergenz 11 zwischen einem Fahrstil des Fahrers 4 und situativen Entscheidungen des Systems reduziert werden kann. Oftmals wird hierfür eine direkte Eingabe des Fahrers 4 zu seinen Wünschen genutzt oder es ist eine größere Menge an Daten erforderlich, um auf Nutzerprofile zu schließen. Dies hat den Nachteil, dass eine händische Parametrierung nur für sehr wenige und einfach verständliche Parameter wie beispielsweise eine Zeitlücke bei Abstandsregelsystemen umsetzbar ist. Darüber hinaus ist eine Fahrerbeobachtung zur Erzeugung von Daten des Nutzerprofils nur sinnvoll bei einer manuellen Fahrt, da in einer assistierten Fahrt sich die Fahrweisen des Fahrers 4 und der Fahrerassistenzfunktion 3 vermischen. Es ist unklar, ob die Daten einer manuellen Fahrt überhaupt dem Wunsch des Fahrers 4 entsprechen, wenn diesem beim Fahren assistiert wird. Genauso wie man als Passagier gegebenenfalls nicht so gefahren werden möchte, wie wenn man selbst fährt, kann es auch im assistierten Modus dazu kommen, dass die Assistierung mit dem eigenen Fahrstil nicht gewünscht sein kann.

Divergieren Assistenzeingriffe von den persönlichen beziehungsweise subjektiven Entscheidungen des nutenden Fahrers 4, kann dies zu einer schlechten Bewertung bis hin zu einem Ausschalten beziehungsweise Nichtnutzen von Fahrerassistenzfunktionen 3 führen. Verhält sich der Fahrer 4 anders als die Fahrerassistenzfunktion 3, dann divergieren die präferierten Aktionen auf Lenkrad, Gas und Bremse voneinander. Bei dem Verfahren zum Generieren der Trainingsdaten 1 ist es vorgesehen, dass diese Divergenz 11 zwischen Fahrer 4 und Fahrerassistenzfunktion 3 als Kennwert, vorliegend als Divergenzwert, quantifiziert wird und diese Divergenz 11 als Input für ein maschinelles Lernverfahren verwendet wird. Sowohl Supervised Learning Verfahren als auch Reinforcement Learning Verfahren können dieses Merkmal der Divergenz 11 als Label oder als negative Rewardfunktion verwenden, um Aktionen der Fahrerassistenzfunktion 3 sukzessive so anzupassen, dass sie zu weniger und seltener zu Divergenz 11 zum Fahrerwunsch führen und durch Berücksichtigung des Systemwunschs der Fahrerassistenzfunktion 3 trotzdem eine Veränderung beziehungsweise eine Verbesserung des Fahrstils des Fahrers 4 bewirkt wird. Dies ermöglicht eine automatisierte Anpassung der Fahrerassistenzfunktion 3, welche zudem auch keine manuelle Fahrt zur Datengewinnung benötigt und darüber hinaus auch weiterlernen kann, wenn der Fahrer 4 sich bereits assistieren lässt. Letzteres ist möglich, da der Softwareagent 2 nicht aus der beobachteten Fahrweise lernt, welche im assistierten Modus eine Mischung aus Fahrer 4 und Fahrerassistenzfunktion 3 ist, sondern aus dem Erkennen von Divergenz 11 beziehungsweise auch durch die nicht vorhandene Divergenz 11 zwischen Fahrer 4 und Fahrerassistenzfunktion 3 lernt. Es kann somit erreicht werden, dass die Fahrerassistenzfunktion 3 durch das Verfahren Schritt für Schritt sich an ideale Assistenzvorstellungen des Fahrers 4 anpasst.

Die Divergenz 11 kann zwischen konkreten Aktivitäten an Lenkrad, Gaspedal und Bremspedal oder als direkter Divergenzwert oder als Divergenz 11 zwischen den Vorstellungen von Fahrerassistenzfunktion 3 und Fahrer 4 als indirekte oder akausale Kennwerte ermittelt werden.

Über Aktionen auf die Schnittstellen des Kraftfahrzeugs können die Fahrerassistenzfunktion 3 und der Fahrer 4 sich gegenseitig wahrnehmen. Eine Divergenz 11 zwischen einer Fahreraktion des Fahrers 4 und einer Systemaktion der Fahrerassistenzfunktion 3 entstehen primär durch unterschiedliche Vorstellungen für die Zukunft. Die Fahrerassistenzfunktion 3 bringt durch unterschiedlich umgesetzte Regelungsparameter in der Aktorikregelung 8 eine zusätzliche Interaktionskomponente mit ein, welche positiv oder negativ bewertet werden kann. Beispiel hierfür ist, wie stark das Moment ist, welches die Fahrerassistenzfunktion 3 maximal nutzen darf, um dem Fahrer 4 zu assistieren.

Die Divergenz 11 kann anhand von Kennwerten basierend auf den schlussendlich ausgeführten Aktionen des Kraftfahrzeugs berechnet werden. Über eine Korrelations- und Kovarianzbildung einer längeren Messreihe können zusätzliche Kennwerte berechnet werden, die für die Adaption zusätzlich zur Bewertung jedes Zeitpunkts genutzt werden können. Anhand der Korrelation des Fahrerlenkmoments und des Assistenzlenkmoments kann unterschieden werden, ob der Fahrer 4 einfach nur passiv das Lenkrad festhält oder aktiv gegen die Empfehlungen der Fahrerassistenzfunktion 3 arbeitet. Ein aktiv eingreifender Fahrer 4 kann die Aktionen der Fahrerassistenzfunktion 3 bestätigen oder diesen entgegenwirken.

Es gibt unterschiedliche Arten, um Divergenzmerkmale zu berechnen. Diese können auch als inverse Akzeptanzmerkmale oder als Widerspruchsbestimmung bezeichnet werden. Diese Divergenzmerkmale können jeden beliebigen Aufbau eines automatisierten Systems adaptieren, insbesondere auch Systeme, welche von Anfang bis Ende aus künstlichen neuronalen Netzen bestehen und keine klar interpretierbare Struktur mehr aufweisen wie in Fig. 2 dargestellt. Die fehlerbezogenen Hirnpotentiale können als zusätzlicher Marker in die Systemadaption eingebracht werden und somit für das Bestimmen der Label der jeweiligen Situationen 5 in den Trainingsdaten 1 herangezogen werden. Der Softwareagent 2 kann ein Vortraining basierend auf bei einer manuellen Fahrt des Kraftfahrzeugs durch den Fahrer 4 gesammelten Daten durchlaufen haben. Einflüsse der Adaption und somit des Anpassens der Fahrerassistenzfunktion 3 durch den Softwareagenten 2 können bei dem Verfahren klar erfasst werden und mit Begrenzungen versehen werden. Insbesondere ist es vorgesehen, dass der Softwareagent 2 lediglich eine Parametrierung der der Fahrerassistenzfunktion 3 unterliegenden Algorithmen adaptiert. Beispielsweise könnte der Softwareagent 2 lernen, dass, wenn er nie eingreift, keine Divergenzen 11 entstehen. Es kann somit für das Anpassen der Fahrerassistenzfunktion 3 beziehungsweise für das Steuern des Kraftfahrzeugs mittels der Fahrerassistenzfunktion 3 vorgegeben werden, dass ein Eingriff der Fahrerassistenzfunktion 3 im Rahmen der Aktorikregelung 8 nicht kleiner sein darf als ein vorgegebener Grenzwert, um diesen Fall zu verhindern.

Das Verfahren ermöglicht, dass die Fahrerassistenzfunktion 3 kontinuierlich und ohne aktive Programmierung des Fahrers 4 an diesen angepasst wird und im laufenden Betrieb des Kraftfahrzeugs immer besser wird.

Insgesamt zeigt die Erfindung, wie eine Nutzung von Divergenzmerkmalen zur Anpassung von lernenden Algorithmen für assistierte Fahrfunktionen umgesetzt werden kann.

### Bezugszeichenliste

- 1: Trainingsdaten
- 2: Softwareagent
- 3: Fahrerassistenzfunktion
- 4: Fahrer
- 5: Situation
- 6: Bahnplanung
- 7: Fahrdynamikregelung
- 8: Aktorikregelung
- 9: Steuereinrichtung
- 10: Ist-Trajektorie
- 11: Divergenz
- 12: fehlerbezogenes Hirnpotential
- 13: Sicherheitsvorhersage
- 14: Eingriffsdominanz
- 15: Anpassungsregelung
- 16: Soll-Trajektorie
- 17: Konvergenzbereich
- 18: Divergenzbereich

## Patentansprüche

1. Verfahren zum Generieren von Trainingsdaten (1) für einen Softwareagenten (2), welcher dazu eingerichtet ist, eine Fahrerassistenzfunktion (3) eines Kraftfahrzeugs zu steuern, bei welchem
- mittels der Fahrerassistenzfunktion (3) für eine ermittelte Situation (5) eine Trajektorie (16) für das Kraftfahrzeug geplant wird, eine für das Führen des Kraftfahrzeugs entlang der Trajektorie (16) vorgesehene Längssteuerung und/oder Quersteuerung geplant wird und für das Umsetzen der geplanten Längssteuerung und/oder Quersteuerung eine Aktorik des Kraftfahrzeugs eingestellt wird,
- eine Reaktion des Fahrers (4) auf die eingestellte Aktorik ermittelt wird,
**dadurch gekennzeichnet, dass**
- in Abhängigkeit von der ermittelten Reaktion festgestellt wird, wie groß eine durch einen Divergenzwert charakterisierte Divergenz (11) einer durch die Reaktion charakterisierten, vom Fahrer geplanten Längssteuerung und/oder Quersteuerung zu der durch die beeinflusste Aktorik charakterisierten, von der Fahrerassistenzfunktion (3) geplanten Längssteuerung und/oder Quersteuerung des Kraftfahrzeugs entlang der Trajektorie (16) ist,
- die Situation gemeinsam mit dem ermittelten zugeordneten Divergenzwert als Trainingsdaten (1) für den Softwareagenten (2) bereitgestellt wird.

2. Verfahren nach Anspruch 1, wobei die Aktorik in Abhängigkeit von einer vorgegebenen Eingriffsdominanz (14) der Fahrerassistenzfunktion (3) eingestellt wird.

3. Verfahren nach Anspruch 1 oder 2, wobei die Reaktion des Fahrers (4) anhand einer Betätigung eines Gaspedals und/oder eines Bremspedals und/oder einer Lenkeinrichtung ermittelt wird.

4. Verfahren nach einem der vorhergehenden Ansprüche, wobei die Reaktion des Fahrers (4) anhand eines gemessenen fehlerbezogenen Hirnpotentials (12) im Gehirn des Fahrers (4) ermittelt wird.

5. Verfahren nach einem der vorhergehenden Ansprüche, wobei der Divergenzwert mit einem vorgegebenen Schwellenwert verglichen wird und in den Trainingsdaten (1) der Situation (5) zugeordnet wird, dass bei der Situation (5) keine Divergenz (11) besteht, wenn festgestellt wird, dass der Divergenzwert kleiner als der vorgegebene Schwellenwert ist, und in den Trainingsdaten (1) der Situation (5) zugeordnet wird, dass eine Divergenz (11) besteht, wenn festgestellt wird, dass der Divergenzwert größer als oder gleich wie der vorgegebene Schwellenwert ist.

6. Verfahren zum Trainieren eines Softwareagenten (2), mittels Trainingsdaten (1), welche in einem Verfahren nach einem der vorhergehenden Ansprüche generiert worden sind.

7. Verfahren nach Anspruch 6, wobei der Softwareagent (2) mittels bestärkendem Lernen trainiert wird, indem eine Häufigkeit einer ermittelten Divergenz (11) oder der Divergenzwert als Belohnung dient und zu minimieren ist.

8. Verfahren zum Assistieren eines Fahrers (4) beim Steuern eines Kraftfahrzeugs mittels einer Fahrerassistenzfunktion (3), bei welchem durch einen Softwareagenten (2), welcher in einem Verfahren nach Anspruch 6 oder 7 trainiert worden ist, eine Fahrerassistenzfunktion (3) angepasst wird und mittels der angepassten Fahrerassistenzfunktion (3) dem Fahrer (4) beim Steuern des Kraftfahrzeugs assistiert wird, indem mittels der Fahrerassistenzfunktion (3) für eine ermittelte Situation (5) eine Aktorik des Kraftfahrzeugs eingestellt wird.

9. Verfahren nach Anspruch 8, wobei durch den Softwareagenten (2) eine Bahnplanung (6) der Fahrerassistenzfunktion (3) und/oder eine Eingriffsdominanz (14) der Fahrerassistenzfunktion (3) angepasst wird und mittels der angepassten Fahrerassistenzfunktion (3) dem Fahrer (4) beim Steuern des Kraftfahrzeugs assistiert wird, indem mittels der Fahrerassistenzfunktion (3) für eine ermittelte Situation (5) eine Trajektorie (16) für das Kraftfahrzeug im Rahmen der angepassten Bahnplanung (6) geplant wird, eine für das Führen des Kraftfahrzeugs entlang der Trajektorie (16) vorgesehene Längssteuerung und/oder Quersteuerung geplant wird und für das Umsetzen der geplanten Längssteuerung und/oder Quersteuerung die Aktorik des Kraftfahrzeugs in Abhängigkeit von der angepassten Eingriffsdominanz (14) eingestellt wird.

10. Kraftfahrzeug mit einem Fahrerassistenzsystem, welches dazu eingerichtet ist, einem Fahrer beim Steuern des Kraftfahrzeugs mittels einer Fahrerassistenzfunktion (3) zu assistieren, welche in einem Verfahren nach Anspruch 8 oder 9 angepasst worden ist.

## Claims

1. Method for generating training data (1) for a software agent (2) which is designed to control a driver assistance function (3) of a motor vehicle, in which method
- by means of the driver assistance function (3), for an ascertained situation (5), a trajectory (16) for the motor vehicle is planned, a longitudinal control and/or lateral control intended for guiding the motor vehicle along the trajectory (16) is planned and an actuator system of the motor vehicle is adjusted in order to implement the planned longitudinal control and/or lateral control,
- a reaction of the driver (4) to the adjusted actuator system is ascertained,
**characterized in that**
- depending on the ascertained reaction, it is established how large a divergence (11), **characterized by** a divergence value, is between a longitudinal control and/or lateral control **characterized by** the reaction and planned by the driver and the longitudinal control and/or lateral control of the motor vehicle along the trajectory (16) that is **characterized by** the influenced actuator system and planned by the driver assistance function (3),
- the situation together with the ascertained assigned divergence value is provided as training data (1) for the software agent (2).

2. Method according to claim 1, wherein the actuator system is adjusted depending on a specified intervention dominance (14) of the driver assistance function (3).

3. Method according to claim 1 or 2, wherein the reaction of the driver (4) is ascertained based on an operation of an accelerator pedal and/or of a brake pedal and/or of a steering device.

4. Method according to any of the preceding claims, wherein the reaction of the driver (4) is ascertained based on a measured error-related brain potential (12) in the brain of the driver (4).

5. Method according to any of the preceding claims, wherein the divergence value is compared with a specified threshold value and in the training data (1) it is assigned to the situation (5) that there is no divergence (11) in the situation (5) if it is established that the divergence value is less than the specified threshold value, and in the training data (1) it is assigned to the situation (5) that there is a divergence (11) if it is established that the divergence value is greater than or equal to the specified threshold value.

6. Method for training a software agent (2) by means of training data (1) which has been generated in a method according to any of the preceding claims.

7. Method according to claim 6, wherein the software agent (2) is trained by means of reinforcement learning, in that a frequency of an ascertained divergence (11) or of the divergence value is used as a reward and is to be minimized.

8. Method for assisting a driver (4) in controlling a motor vehicle by means of a driver assistance function (3), in which method a driver assistance function (3) is adapted by a software agent (2) which has been trained in a method according to claim 6 or 7 and the driver (4) is assisted in controlling the motor vehicle by means of the adapted driver assistance function (3), in that an actuator system of the motor vehicle is adjusted for an ascertained situation (5) by means of the driver assistance function (3).

9. Method according to claim 8, wherein a path planning (6) of the driver assistance function (3) and/or an intervention dominance (14) of the driver assistance function (3) is adapted by the software agent (2) and the driver (4) is assisted in controlling the motor vehicle by means of the adapted driver assistance function (3), in that by means of the driver assistance function (3), for an ascertained situation (5), a trajectory (16) for the motor vehicle is planned within the framework of the adapted path planning (6), a longitudinal control and/or lateral control intended for guiding the motor vehicle along the trajectory (16) is planned and the actuator system of the motor vehicle is adjusted depending on the adapted intervention dominance (14) in order to implement the planned longitudinal control and/or lateral control.

10. Motor vehicle comprising a driver assistance system which is designed to assist a driver in controlling the motor vehicle by means of a driver assistance function (3) which has been adapted in a method according to claim 8 or 9.

## Revendications

1. Procédé pour la génération de données d'apprentissage (1) pour un agent logiciel (2) qui est configuré pour commander une fonction d'aide à la conduite (3) d'un véhicule automobile, dans lequel
- une trajectoire (16) pour le véhicule automobile est planifiée à l'aide de la fonction d'aide à la conduite (3) pour une situation (5) déterminée, une commande longitudinale et/ou une commande transversale prévues pour le guidage du véhicule automobile le long de la trajectoire (16) sont planifiées, et un système d'actionneurs du véhicule automobile est réglé pour la mise en œuvre de la commande longitudinale et/ou de la commande transversale planifiées,
- une réaction du conducteur (4) face au réglage du système d'actionneurs est déterminée, **caractérisé en ce que**
- en fonction de la réaction déterminée, on constate l'ampleur d'une divergence (11) **caractérisée par** une valeur de divergence d'une commande longitudinale et/ou d'une commande transversale, **caractérisées par** la réaction et planifiées par le conducteur, par rapport à la commande longitudinale et/ou à la commande transversale du véhicule automobile, **caractérisées par** le système d'actionneurs influencé et planifiées par la fonction d'aide à la conduite (3), le long de la trajectoire (16),
- la situation, conjointement avec la valeur de divergence associée et déterminée, sont fournies en tant que données d'entraînement (1) pour l'agent logiciel (2).

2. Procédé selon la revendication 1, dans lequel le système d'actionneurs est réglé en fonction d'une dominance d'intervention (14) prédéfinie de la fonction d'aide à la conduite (3).

3. Procédé selon la revendication 1 ou 2, dans lequel la réaction du conducteur (4) est déterminée à partir de l'actionnement d'une pédale d'accélérateur et/ou d'une pédale de frein et/ou d'un dispositif de direction.

4. Procédé selon l'une des revendications précédentes, dans lequel la réaction du conducteur (4) est déterminée à partir d'un potentiel cérébral lié à une erreur (12) mesuré dans le cerveau du conducteur (4).

5. Procédé selon l'une des revendications précédentes, dans lequel la valeur de divergence est comparée avec une valeur seuil prédéfinie et est associée, dans les données d'apprentissage (1), à la situation (5) selon laquelle, dans la situation (5), il n'existe pas de divergence (11) lorsqu'il est constaté que la valeur de divergence est inférieure à la valeur seuil prédéfinie, et est associée, dans les données d'apprentissage (1), à la situation (5) selon laquelle il existe une divergence (11) lorsqu'il est constaté que la valeur de divergence est supérieure ou égale à la valeur seuil prédéfinie.

6. Procédé pour l'entraînement d'un agent logiciel (2) à l'aide de données d'entraînement (1) qui ont été générées dans un procédé selon l'une des revendications précédentes.

7. Procédé selon la revendication 6, dans lequel l'agent logiciel (2) est entraîné à l'aide d'un apprentissage par renforcement par le fait que la fréquence d'une divergence (11) déterminée ou la valeur de divergence sert de récompense et doit être minimisée.

8. Procédé permettant d'aider un conducteur (4) lors de la conduite d'un véhicule automobile au moyen d'une fonction d'aide à la conduite (3), dans lequel une fonction d'aide à la conduite (3) est adaptée par un agent logiciel (2) qui a été entraîné dans un procédé selon la revendication 6 ou 7, et, au moyen de la fonction d'aide à la conduite (3) adaptée, le conducteur (4) est aidé lors de la conduite du véhicule automobile par le fait qu'au moyen de la fonction d'aide à la conduite (3), un système d'actionneurs du véhicule automobile est réglé pour une situation (5) déterminée.

9. Procédé selon la revendication 8, dans lequel, au moyen de l'agent logiciel (2), une planification de trajectoire (6) de la fonction d'aide à la conduite (3) et/ou une priorité d'intervention (14) de la fonction d'aide à la conduite (3) sont adaptées et, au moyen de la fonction d'aide à la conduite (3) adaptée, le conducteur (4) est aidé lors de la conduite du véhicule automobile par le fait que, au moyen de la fonction d'aide à la conduite (3), une trajectoire (16) est planifiée pour le véhicule automobile pour une situation déterminée dans le cadre de la planification de trajectoire (6) adaptée, une commande longitudinale et/ou une commande transversale prévues pour le guidage du véhicule automobile le long de la trajectoire (16) sont planifiées, et, pour la mise en œuvre de la commande longitudinale et/ou de la commande transversale planifiées, le système d'actionneurs du véhicule est réglé en fonction de la dominance d'intervention (14) adaptée.

10. Véhicule automobile comportant un système d'aide à la conduite qui est configuré pour aider un conducteur lors de la conduite du véhicule automobile au moyen d'une fonction d'aide à la conduite (3) qui a été adaptée dans un procédé selon la revendication 8 ou 9.
